# EUROPEAN PATENT SPECIFICATION

(11) **EP 1 910 058 B1**
(45) Date of publication and mention of the grant of the patent: **20.04.2011**
(21) Application number: 06779860.3
(22) Date of filing: 13.07.2006
(51) Int. Cl.: B29C 49/20, B29C 49/48, B29C 49/04

(54) **MACHINE AND METHOD FOR CARRYING OUT A CONTAINER AND CONTAINER OBTAINED THEREBY**
MASCHINE UND VERFAHREN ZUR HERSTELLUNG EINES BEHÄLTERS UND DADURCH HERGESTELLTER BEHÄLTER
MACHINE ET PROCEDE DESTINES A PRODUIRE UN CONTENEUR ET CONTENEUR AINSI OBTENU

(30) Priority: 14.07.2005 IT BO20050468
(43) Date of publication of application: 16.04.2008
(73) Proprietor: Brevetti Angela S.r.L., 36071 Arzignano (IT)
(72) Inventor: CONSOLARO, Roberto, I-36071 Arzignano (IT); CONSOLARO, Angelo, I-36071 Arzignano (IT)
(74) Representative: Iannone, Carlo Luigi
(86) International application number: PCT/IB2006/001935
(87) International publication number: WO 2007/007178

(56) References cited:
- WO-A-01/43799
- DE-A1- 10 245 318
- DE-A1- 19 518 426
- US-A- 5 687 550
- US-B1- 6 585 693

## Description

### TECHNICAL FIELD

The present invention relates to the field of containers and it refers to a machine and a method to carry out a container and the container obtained thereby. In particular the container can be of mono-dose or multi-dose type for liquid and provided with accessories such as injection needles, connectors or others.

### BACKGROUND ART

There are known machines and methods for the production of plastic mono-dose vials, each of them is filled with a liquid and provided with a needle for the injection of such liquid.

Said needle is inserted by force in a plastic element and it is provided with a protection means to maintain the sterility and the sharpening of the point.

After the container is filled, the plastic element is inserted in the filling mouthpiece of the container and then is sealed in a manner that the needle sticks out from the container and it is in flow communication with the inside of said container.

A drawback of said known machines and methods consists in that they need a lot of production phases, they are complex, expensive and they can not always provide optimal results.

Further drawback consists in that the connection between the plastic element of the needle and the container is critical and it can cause limitations of use.

Other drawback consists in the fact that the protection means of the needle of said known containers could be difficult to be removed or, on the contrary, can be not enough to assure the necessary sterility and protection.

Document DE10245318A1 discloses a machine for carrying out a container for a product and provided with a respective accessory, starting from a tubular element of semi-fluid plastic material. Said machine comprises production means of the tubular element of semi-fluid plastic material; moulding means fed by the production means of the tubular element and driven between closing and opening positions; moulding forming means adjacent to the moulding means (2) driven between closing and opening positions; support means suitable for positioning the accessory into the forming mean; filling means suitable for supplying the product (P).

### DISCLOSURE OF THE INVENTION

An object of the present invention is to propose a machine and a method for carrying out a container equipped with an accessory, that they are simple, provided with a small quantity of components and of production phases and that they provide reliable results. Other object is to propose a container equipped with a connection with its own accessory, for instance a needle, very simple, reliable and with a very good mechanical resistance and liquid sealed.

Further object is to propose a container whose connection to the accessory occurs at low temperatures and without excessive emission of volatile substances and so that it dose not cause significant limitations of use of the said container.

Other object is to propose a container carried out in integral with the protection means of the needle.

Further object is to propose a container in which the protection means assures the sterility and the protection of the sharpening of the needle and that, at the same time, it is easy to be removed by an user.

The objects of the invention are solved by apparatus claim 1, method claim 10 and product claim 14. Further embodiments are the subject of the dependent claims.

### BRIEF DESCRIPTION OF THE DRAWINGS

The characteristics of the invention are highlighted in the following with particular reference to the attached drawings, in which:
- figures 1 - 8 show schematic and partial transversal sectioned views of the machine for making a container in a sequence of production phases of the container according to the method object of the present invention;
- figure 9 shows a side view of the container of figure 8 separated from the off-cuts;
- figures 10 and 11 show side views of the container of figure 9 with the protection means of the needle respectively assembled and removed;
- figures 12 and 13 show respectively enlarged side view arid front view of the container of figure 9.

### BEST MODE OF CARRYING OUT THE INVENTION

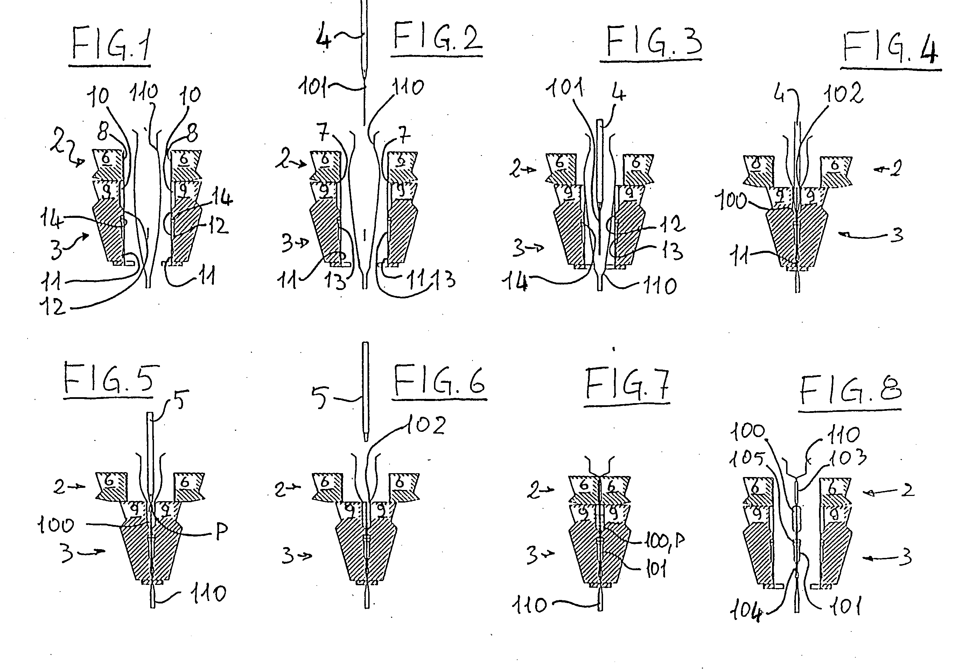

With reference to the figures 1 - 8, numeral 1 indicates the machine object of the present invention to carry out a container, starting from at least a semi-fluid plastic tubular element 110, a container 100 provided of a respective accessory 101 for example consisting of a needle for injections, and suitable for containing a product P, for example a dose of an injectable drug.

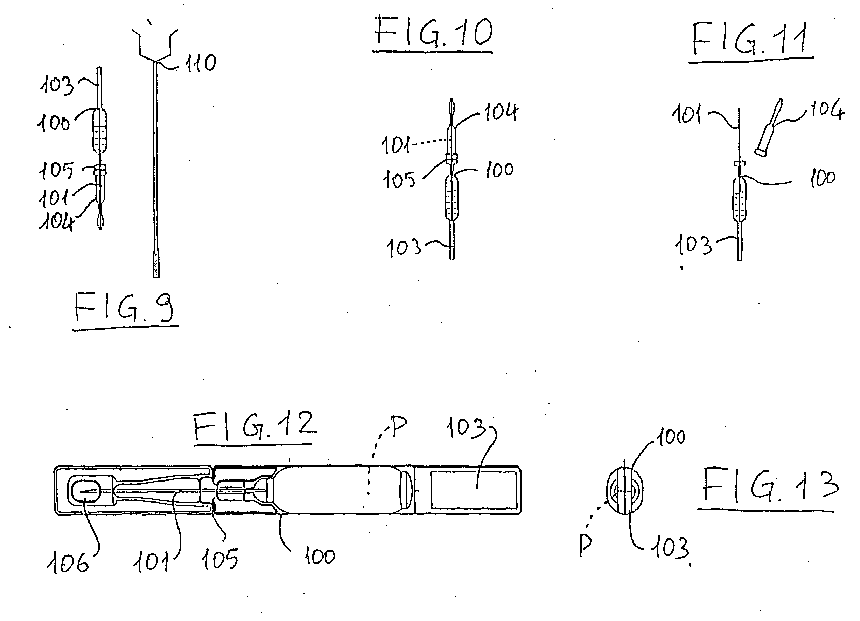

Said container is shown in figures 9 - 13 and the respective plastic material is preferably transparent or semitransparent.

The machine 1 comprises production means of the semi-fluid plastic tubular element 110. Said production means, of know type and not shown, can consist, for example, in a so-called "blow moulding" device for the formation of the said semi-fluid tubular element so-called "parison". Said device can be equipped with upper blowing means to control the internal pressure of the "parison". The machine comprises furthermore moulding means 2 that, in an operational position of the machine 1, they are fed by the production means of the tubular element and are driven between closing and opening positions; forming means 3 are adjacent and nearly coaxial to the moulding means 2 and driven between closing and opening positions.

The machine is also equipped with support means 4, suitable for moving the accessory 101 between internal and external positions to the forming mean 3 and to release it in the internal position, and with filling means 5 moving between internal and external positions in respect to the forming means 3 and suitable for supplying the product P.

Said support means 4 and filling means 5 can be constituted by separate elements or, in alternative, they can be constituted by an unique element of elongated tubular shape having a removable taking means for the accessory 101 and a supply for the product P.

The moulding means 2 and forming means 3 are in mutual contact and their lower parts are aligned up in respect to the production means of the tubular element, to the support means 4 and filling means 5.

In the operational position, the production means feed the moulding means 2 and the forming means 3 in the opening position with the tubular element 110; the support means insert the accessory 101 into the tubular element 110 in the forming means 3.

Said forming means 3, passing into the closing position, form the container 100, with an open end 102, and incorporate in the wall of said container 100 an end portion of the accessory 101 in a manner that it flows in the portion of the container suitable for containing the product P and that, at the same time, sticks out from said portion.

The filling means 5 feed said portion with the product P through the open end 102 of the container.

After the removal of the filling means 5, the moulding means 2, passing into the closing position close the opening 102 of the container 100; the return of the moulding means 2 and of the forming means 3 to the opening condition, renders available the container 100.

The moulding means 2 include two respective moving elements 6, each provided with a first compression means 7 for the closure of the open end 102 of the container 100 and with first moulding means 8 for the moulding of a grip 103 of the container 100.

Also the forming means 3 include two respective moving elements 9, each provided with respective second moulding means 10 and with second compression means 11 respectively for the forming of the portion of the container suitable for containing the product P and for the closure of the end of the container 100 opposite in respect to the open end 102.

Besides, the moving elements 9 of the forming means 3 have respective embedding means 12 adjacent to the second moulding means 10 and suitable for compressing the semi-fluid plastic of the tubular element 110 against the portion of the accessory 101 flowing in the portion for the product, to embed it.

The moving elements 9 of the forming means 3 comprise also respective third moulding means 13 suitable for forming a protection means 104 for the accessory 101 and respective fourth moulding means 14 interposed between the embedding means 12 and the third moulding means 13 and suitable for forming a separation means 105 for the separation of the protection means 104 from the container 100.

The operation of the machine 1 provides the phases:
- to carry out a semi-fluid plastic tubular element 110 by means of production means;
- to insert the tubular element 110 into moulding means 2 and forming means 3 in an opening position thereof;
- to insert an accessory 101 by means of support means 4, inside the tubular element 110, into the forming means 3;
- to set the forming means 3 in a respective closing position forming the container 100 with an open end 102 and embedding the accessory 101 therein;
- to take out at least partially the support means 4 by detaching it from the accessory,
- to feed the container 100 through its open end 102 with the product P by means of filling means 5;
- to remove the filling means 5;
- to set the moulding means 2 in a respective closing position, closing the open end 102 of the container 100;
- to wait for the consolidation of the material of the tubular element 110;
- to set the moulding means 2 and forming means 3 in the opening position and to take out the formed container 100 with the embedded accessory 101.

Furthermore, the method comprises the phases to separate the container 100 formed by the off-cuts of the tubular element 110; to maintain the internal pressure of the tubular element 110 higher then the external pressure by means of the blowing means, or in alternative by means of known but not shown external depression means, and to reduce the internal pressure of the tubular element 110 before setting the moulding means 2 into the respective closing position to avoid excesses of pressure inside the container.

The container comprises a hollow portion containing the product P, an accessory 101 at least partially embedded in the container and flowing into the hollow portion and a grip 103, having almost rectangular and flattened shape, carried out in integral with the container 100 to the end of this latter opposite to the accessory 101.

The accessory 101 can consist, for example, in a fitting or in a connection for the liquid product P or, preferably it consists in a needle for injections as shown in the drawings. The container comprises a protection means 104, integral with the remaining parts of the container 100, for the portion of the accessory 101 protruding from the hollow portion for the product P of the container. Said protection means 104 is fixed to the remaining part of the container by means of a separation means 105 of the protection means 104 and that it has a closed line where the material of the container is thinned for allowing the user to separate the protection means 104 without force.

The portion of the needle 101 incorporated in the material of the container, is provided with relieves and/or depressions and/or grooves and/or knurling and similar suitable for increasing the fixing tenacity of the needle to the material of the container.

The portion of the protection means 104 corresponding to the point of the needle 101 is provided with a swelling 106 fit to guarantee a better protection and to verify that the needle is not obstructed.

An advantage of the present invention is provide a machine and a method to make a container provided with an accessory, that they are simple, provided with a small quantity of components and of phases and with reliable results.

Other advantage is to provide a container equipped with a connection with its own accessory, for example a needle, extremely simple, reliable and with very good mechanical resistance and liquid sealed.

Further advantage is to provide a container whose connection to the accessory occurs at low temperatures and without excessive emissions of volatile substances and that it doesn't cause significant limitations of use of the container.

Other advantage is to provide a container carried out integral with the protection means of the needle to assure the sterility and the protection of the sharpening of said needle and that, at the same time, it is easy to be removed by an user.

## Claims

1. Machine (1) for carrying out, starting from at least a tubular element (110) of semi-fluid plastic material, at least a container (100) for a product (P) and provided with a respective accessory (101), said machine comprising at least:
- production means of the tubular element (110) of semi-fluid plastic material;
- moulding means (2) that, in an operational condition of the machine (1), are fed by the production means of the tubular element and driven between closing and opening positions;
- forming means (3) adjacent and nearly coaxial to the moulding means (2) and driven between closing and opening positions;
- support means (4) suitable for moving the accessory (101) between internal and external positions in respect to the forming means (3) and to release the accessory (101) in said external position;
- filling means (5) moving between internal and external positions in respect to the forming means (3) and suitable for supplying the product (P),
**characterized in that** there are means provided for driving the forming means (3) one towards the other to said closing position in such a way as to form said container (100) with an open end (102) for filling said product (P) and a closed end, wherein the tubular element (110) is being compressed at the closed end directly onto a portion of said accessory (101) to partly embed said accessory (101) into said container (100).

2. Machine (1) according to claim 1 **characterized in that** the moulding means (2) and the forming means (3) are in mutual contact and their lower parts are aligned up in respect to the production means of the tubular element, the support means (4) and the filling means (5).

3. Machine (1) according to claim 1 **characterized in that** the moulding means (2) have at least two respective moving elements (6), each provided with at least a first compression mean (7) for closing the open end (102) of the container (100).

4. Machine (1) according to claim 3 **characterized in that** the moving elements (6) of the moulding means (2) include respective first moulding means (8) for the moulding of a grip (103) of the container (100).

5. Machine (1) according to claim 1 **characterized in that** the forming means (3) have at least two respective moving elements (9), each provided with respective second moulding means (10) and with second compression means (11) respectively for the forming of the portion of the container (100) fit to contain the product (P) and for closing the end of the container (100) opposite in respect to the open end (102).

6. Machine (1) according to claim 5 **characterized in that** the moving elements (9) of the forming means (3) have respective embedding means (12) adjacent to the second moulding means (10) and fit to compress the semi-fluid plastic material of the tubular element (110) against a portion of the accessory (101) embedding it.

7. Machine (1) according to claim 6 **characterized in that** the moving elements (9) of the forming means (3) have respective third moulding means (13) fit to form a protection means (104) for the accessory (101).

8. Machine (1) according to claim 7 **characterized in that** the moving elements (9) of the forming means (3) have respective fourth moulding means (14) interposed between the embedding means (12) and the third moulding means (13) and suitable for forming a separation means (105) in order to allow the manual separation of the protection means (104) from the container (100).

9. Machine (1) according to claim 1 **characterized in that** the support means (4) and the filling means (5) are constituted by an unique elongated tubular shaped element having a removable taking mean for the accessory (101) and a supply for the product (P).

10. Method for making a container by means of the machine of any one of the preceding claims comprising the following phases:
- providing a tubular element (110) of semi-fluid plastic material by means of production means;
- inserting the tubular element (110) in moulding means (2) and in forming means (3) in an opening position;
- inserting an accessory (101) by means of support means (4), inside the tubular element (110), in the forming means (3);
- setting the forming means (3) in a respective closing position forming the container (100) with an open end (102) and a closed end and embedding therein the accessory (101);
- taking out at least partially the support means (4) by detaching it from the accessory (101);
- feeding the container (100) through its open end (102) with the product (P) by means of filling means (5);
- removing the filling means (5);
- setting the moulding means (2) in a respective closing position, closing the open end (102) of the container (100);
- waiting for the consolidation of the material of the tubular element (110);
- setting the moulding means (2) and forming means (3) in the opening position and taking out the container (100) formed,
**characterized in that** said forming means (3) are driven one towards the other to said closing position in such a way as to form said container (100) with the open end for filling the product (P) and to compress the tubular element (110) at the closed end directly onto a portion of said accessory (101) to partly embed said accessory (101) into said container at the closed end.

11. Method according to claim 10 **characterized in that** it separates the formed container (100) from the out-cuts of the tubular element (110).

12. Method according to claim 10 **characterized in that** it maintains the internal pressure of the tubular element (110) higher that the external pressure by means of blowing means or external depression means.

13. Method according to claim 12 **characterized in that** it reduces the internal pressure of the tubular element (110) before setting the moulding means (2) in the respective closing position.

14. Container (100) carried out by means of the machine according to any one of the claims from 1 to 9 or by means of the method according to any one of claims from 10 to 13 comprising at least a hollow portion, containing a product (P) to be dispensed, and an accessory (101) at least partially embedded in the hollow portion and flowing in the hollow portion, **characterized in that** an end portion of said accessory (101) is incorporated or embedded directly into the wall of said container (100) through the compressed plastic material of said container (100).

15. Container (100) according to claim 14 **characterized in that** it comprises a grip (103) carried out integral with the container (100) at the end of said container (100) opposite in respect to the accessory (101).

16. Container (100) according to claim 15 **characterized in that** the grip (103) is shaped almost rectangular and flattened.

17. Container (100) according to claim 14 **characterized in that** it comprises a protection means (104) for the portion of the accessory (101) protruding from the portion for the product (P).

18. Container (100) according to claim 17 **characterized in that** the protection means (104) is fixed to the remaining part of the container (100) by means of a separation means (105) of the protection means (104).

19. Container (100) according to claim 18 **characterized in that** the separation means (105) has a closed line where the material of the container is thinned.

20. Container (100) according to claim 17 **characterized in that** the protection means (104) is integral with the remaining part of the container (100).

21. Container (100) according to claim 14 **characterized in that** the accessory (101) is a needle for injections.

22. Container (100) according to claim 21 **characterized in that** the portion of the needle (101) embedded in the material of the container (100) is provided with at least one among relieves, depressions, grooves, knurling and similar.

23. Container (100) according to any of the claims from 17 to 21 **characterized in that** the portion of the protection means (104) corresponds to the point of the needle (101) which has a swelling (106).

## Patentansprüche

1. Maschine (1) zum Herstellen mindestens eines mit einem entsprechenden Zubehörteil (101) ausgestatteten Behälters (100) für ein Produkt (P), ausgehend von mindestens einem röhrenförmigen Element (110) aus halbflüssigem Kunststoffmaterial, wobei die Maschine mindestens Folgendes umfasst:
- Produktionsmittel des röhrenförmigen Elements (110) aus halbflüssigem Kunststoffmaterial;
- Formgebungsmittel (2), die in einem Betriebszustand der Maschine (1) mit dem röhrenförmigen Element durch das Produktionsmittel beschickt und zwischen Öffnungs- und Schließpositionen angetrieben werden,
- Umformungsmittel (3), die an die Formgebungsmittel (2) angrenzen und nahezu koaxial zu diesen sind und zwischen Öffnungs- und Schließpositionen angetrieben werden;
- Trägermittel (4), die geeignet sind, das Zubehörteil (101) in Bezug auf die Umformungsmittel (3) zwischen inneren und äußeren Positionen zu bewegen und das Zubehörteil (101) in der äußeren Position freizugeben;
- Füllmittel (5), die sich in Bezug auf die Umformungsmittel (3) zwischen inneren und äußeren Positionen bewegen und geeignet sind, das Produkt (P) zuzuführen,
**dadurch gekennzeichnet, dass** Mittel zum Antreiben der Umformungsmittel (3) aufeinander zu in die Schließposition derart bereitgestellt sind, dass der Behälter (100) mit einem offenen Ende (102) zum Befüllen mit dem Produkt (P) und einem geschlossenen Ende gebildet wird, wobei das röhrenförmige Element (110) am geschlossenen Ende direkt auf einen Abschnitt des Zubehörteils (101) gepresst wird, um das Zubehörteil (101) teilweise in den Behälter (100) einzubetten.

2. Maschine (1) nach Anspruch 1, **dadurch gekennzeichnet, dass** die Formgebungsmittel (2) und die Umformungsmittel (3) in gegenseitigem Kontakt stehen und ihre unteren Teile in Bezug auf die Produktionsmittel des röhrenförmigen Elements, die Trägermittel (4) und die Füllmittel (5) ausgerichtet sind.

3. Maschine (1) nach Anspruch 1, **dadurch gekennzeichnet, dass** die Formgebungsmittel (2) mindestens zwei entsprechende sich bewegende Elemente (6) aufweisen, von denen jedes mit mindestens einem Pressmittel (7) zum Schließen des offenen Endes (102) des Behälters (100) ausgestattet ist.

4. Maschine (1) nach Anspruch 3, **dadurch gekennzeichnet, dass** die sich bewegenden Elemente (6) der Formgebungsmittel (2) entsprechende erste Formgebungsmittel (8) zum Formen eines Griffes (103) des Behälters (100) aufweisen.

5. Maschine (1) nach Anspruch 1, **dadurch gekennzeichnet, dass** die Umformungsmittel (3) mindestens zwei entsprechende sich bewegende Elemente (9) aufweisen, die jeweils mit entsprechenden zweiten Formgebungsmitteln (10) und entsprechend mit zweiten Pressmitteln (11) ausgestattet sind, um den Abschnitt des Behälters (100) zu bilden, der geeignet ist, das Produkt (P) zu enthalten, und um das dem offenen Ende (102) gegenüber liegende Ende des Behälters (100) zu verschließen.

6. Maschine (1) nach Anspruch 5, **dadurch gekennzeichnet, dass** die sich bewegenden Elemente (9) der Umformungsmittel (3) entsprechende Einbettungsmittel (12) aufweisen, die an die zweiten Formgebungsmittel (10) angrenzen und geeignet sind, das halbflüssige Kunststoffmaterial des röhrenförmigen Elements (110) gegen einen Abschnitt des Zubehörteils (101) zu pressen und diesen einzubetten.

7. Maschine (1) nach Anspruch 6, **dadurch gekennzeichnet, dass** die sich bewegenden Elemente (9) der Umformungsmittel (3) entsprechende dritte Formgebungsmittel (13) aufweisen, die geeignet sind, ein Schutzmittel (104) für das Zubehörteil (101) zu bilden.

8. Maschine (1) nach Anspruch 7, **dadurch gekennzeichnet, dass** die sich bewegenden Elemente (9) der Umformungsmittel (3) entsprechende vierte Formgebungsmittel (14) aufweisen, die zwischen den Einbettungsmitteln (12) und den dritten Formgebungsmitteln (13) angeordnet und geeignet sind, ein Abtrennmittel (105) zu bilden, um das manuelle Abtrennen der Schutzmittel (104) vom Behälter (100) zu ermöglichen.

9. Maschine (1) nach Anspruch 1, **dadurch gekennzeichnet, dass** die Trägermittel (4) und die Füllmittel (5) aus einem einzigen länglichen röhrenförmigen Formelement gebildet sind, das ein entfernbares Aufnahmemittel für das Zubehörteil (101) und eine Zufuhr für das Produkt (P) aufweist.

10. Verfahren zur Herstellung eines Behälters mit Hilfe der Maschine nach einem der vorhergehenden Ansprüche, folgende Phasen umfassend:
- Bereitstellen eines röhrenförmigen Elements (110) aus halbflüssigem Kunststoffmaterial als Produktionsmittel,
- Einführen des röhrenförmigen Elements (110) in Formgebungsmittel (2) und Umformungsmittel (3) in einer Öffnungsposition,
- Einführen eines Zubehörteils (101) in das Innere des röhrenförmigen Elements (110) in den Umformungsmitteln (3) mit Hilfe von Trägermitteln (4),
- Einstellen der Umformungsmittel (3) in eine entsprechende Schließposition, wobei der Behälter (100) mit einem offenen Ende (102) und einem geschlossenen Ende gebildet und das Zubehörteil darin eingebettet wird,
- mindestens teilweises Herausnehmen der Trägermittel (4) durch Loslösen vom Zubehörteil (101),
- Beschicken des Behälters (100) durch sein offenes Ende (102) hindurch mit dem Produkt (P) mit Hilfe von Füllmitteln (5),
- Entfernen der Füllmittel (5),
- Einstellen der Formgebungsmittel (2) in eine entsprechende Schließposition, Schließen des offenen Endes (102) des Behälters (100),
- Warten auf die Verfestigung des Materials des röhrenförmigen Elements (110),
- Einstellen der Formgebungsmittel (2) und Umformungsmittel (3) in die Öffnungsposition und Herausnehmen des gebildeten Behälters (100),
**dadurch gekennzeichnet, dass** die Umformungsmittel (3) derart aufeinander zu in die Schließposition angetrieben werden, dass der Behälter (100) mit dem offenen Ende (102) zum Befüllen mit dem Produkt (P) gebildet wird und das röhrenförmige Element (110) am geschlossenen Ende direkt auf einen Abschnitt des Zubehörteils (101) gepresst wird, um das Zubehörteil (101) am geschlossenen Ende teilweise in den Behälter (100) einzubetten.

11. Verfahren nach Anspruch 10, **dadurch gekennzeichnet, dass** es den gebildete Behälter (100) von den Abschnitten des röhrenförmigen Elements (110) trennt.

12. Verfahren nach Anspruch 10, **dadurch gekennzeichnet, dass** es den Innendruck im röhrenförmigen Element (110) mit Hilfe von Blasmitteln oder externen Dekompressionsmitteln höher als den Außendruck hält.

13. Verfahren nach Anspruch 12, **dadurch gekennzeichnet, dass** es den Innendruck im röhrenförmigen Element (110) vor dem Einstellen der Formgebungsmittel (2) in die entsprechende Schließposition vermindert.

14. Behälter (100), ausgeführt mit Hilfe der Maschine nach einem der Ansprüche 1 bis 9 oder mit Hilfe des Verfahrens nach einem der Ansprüche 10 bis 13, umfassend mindestens einen hohlen Abschnitt, der ein abzugebendes Produkt (P) enthält, und ein Zubehörteil (101), das mindestens teilweise in den hohlen Abschnitt eingebettet ist und sich in den hohlen Abschnitt erstreckt, **dadurch gekennzeichnet, dass** ein Endabschnitt des Zubehörteils (101) durch das verpresste Kunststoffmaterial des Behälters (100) direkt in die Wandung des Behälters (100) integriert oder eingebettet ist.

15. Behälter (100) nach Anspruch 14, **dadurch gekennzeichnet, dass** er einen Griff (103) umfasst, der am Ende des Behälters (100) gegenüber dem Zubehörteil (101) einstückig mit dem Behälter (100) ausgeführt ist.

16. Behälter (100) nach Anspruch 15, **dadurch gekennzeichnet, dass** der Griff (103) nahezu rechteckig und abgeflacht geformt ist.

17. Behälter (100) nach Anspruch 14, **dadurch gekennzeichnet, dass** er ein Schutzmittel (104) für den Abschnitt des Zubehörteils (101) umfasst, der aus dem Abschnitt für das Produkt (P) hervorsteht.

18. Behälter (100) nach Anspruch 17, **dadurch gekennzeichnet, dass** das Schutzmittel (104) mit Hilfe eines Abtrennmittels (105) des Schutzmittels (104) am verbleibenden Teil des Behälters (100) befestigt ist.

19. Behälter (100) nach Anspruch 18, **dadurch gekennzeichnet, dass** das Abtrennmittel (105) eine durchgehende Linie aufweist, an der das Material des Behälters dünner gehalten ist.

20. Behälter (100) nach Anspruch 17, **dadurch gekennzeichnet, dass** das Schutzmittel (104) einstückig mit dem verbleibenden Teil des Behälters (100) gebildet ist.

21. Behälter (100) nach Anspruch 14, **dadurch gekennzeichnet, dass** das Zubehörteil (101) eine Injektionsnadel ist.

22. Behälter (100) nach Anspruch 21, **dadurch gekennzeichnet, dass** der in das Material des Behälters (100) eingebettete Abschnitt der Nadel (101) mit mindestens einem der Folgenden ausgestattet ist: Aussparungen, Vertiefungen, Rillen, Rändelungen und dergleichen.

23. Behälter (100) nach einem der Ansprüche 17 bis 21, **dadurch gekennzeichnet, dass** der Abschnitt des Schutzmittels (104) der Spitze der Nadel (101) entspricht, die eine Wölbung (106) aufweist.

## Revendications

1. Machine (1) pour la réalisation, à partir d'au moins un élément tubulaire (110) en matière plastique semi-fluide, d'au moins un conteneur (100) pour un produit (P) et pourvu d'un accessoire respectif (101), ladite machine comprenant au moins :
- des moyens de production de l'élément tubulaire (110) en matière plastique semi-fluide ;
- des moyens de moulage (2) qui, dans un état de fonctionnement de la machine (1), sont alimentés par les moyens de production de l'élément tubulaire et actionnés entre des positions de fermeture et d'ouverture ;
- des moyens de formation (3) adjacents et presque coaxiaux aux moyens de moulage (2) et actionnés entre des positions de fermeture et d'ouverture ;
- des moyens de support (4) appropriés pour déplacer l'accessoire (101) entre des positions interne et externe par rapport aux moyens de formation (3) et pour relâcher l'accessoire (101) dans ladite position externe ;
- des moyens de remplissage (5) mobiles entre des positions interne et externe par rapport aux moyens de formation (3) et appropriés pour amener le produit (P),
**caractérisée en ce que** des moyens sont prévus pour actionner les moyens de formation (3) l'un vers l'autre jusqu'à ladite position de fermeture, de façon à former ledit conteneur (100) avec une extrémité ouverte (102) pour le remplissage dudit produit (P) et une extrémité fermée, l'élément tubulaire (110) étant comprimé, au niveau de l'extrémité fermée, directement sur une partie dudit accessoire (101) de façon à incorporer partiellement ledit accessoire (101) dans ledit conteneur (100).

2. Machine (1) selon la revendication 1, **caractérisée en ce que** les moyens de moulage (2) et les moyens de formation (3) sont en contact mutuel et leurs parties inférieures sont alignées par rapport aux moyens de production de l'élément tubulaire, aux moyens de support (4) et aux moyens de remplissage (5).

3. Machine (1) selon la revendication 1, **caractérisée en ce que** les moyens de moulage (2) ont au moins deux éléments mobiles respectifs (6), qui sont chacun pourvus d'au moins un premier moyen de compression (7) pour la fermeture de l'extrémité ouverte (102) du conteneur (100).

4. Machine (1) selon la revendication 3, **caractérisée en ce que** les éléments mobiles (6) des moyens de moulage (2) comprennent des premiers moyens de moulage respectifs (8) pour le moulage d'un organe de préhension (103) du conteneur (100).

5. Machine (1) selon la revendication 1, **caractérisée en ce que** les moyens de formation (3) ont au moins deux éléments mobiles respectifs (9), qui sont chacun pourvus de deuxièmes moyens de moulage respectifs (10) et de deuxièmes moyens de compression (11) pour, respectivement, la formation de la partie du conteneur (100) destinée à contenir le produit (P) et la fermeture de l'extrémité du conteneur (100) opposée à l'extrémité ouverte (102).

6. Machine (1) selon la revendication 5, **caractérisée en ce que** les éléments mobiles (9) des moyens de formation (3) ont des moyens d'incorporation respectifs (12) adjacents aux deuxièmes moyens de moulage (10) et destinés à comprimer la matière plastique semi-fluide de l'élément tubulaire (110) contre une partie de l'accessoire (101), incorporant ainsi ce dernier.

7. Machine (1) selon la revendication 6, **caractérisée en ce que** les éléments mobiles (9) des moyens de formation (3) ont des troisièmes moyens de moulage respectifs (13) destinés à former un moyen de protection (104) pour l'accessoire (101).

8. Machine (1) selon la revendication 7, **caractérisée en ce que** les éléments mobiles (9) des moyens de formation (3) ont des quatrièmes moyens de moulage respectifs (14) intercalés entre les moyens d'incorporation (12) et les troisièmes moyens de moulage (13) et appropriés pour former un moyen de séparation (105) afin de permettre la séparation manuelle du moyen de protection (104) d'avec le conteneur (100).

9. Machine (1) selon la revendication 1, **caractérisée en ce que** les moyens de support (4) et les moyens de remplissage (5) sont constitués d'un unique élément de forme tubulaire allongée ayant des moyens de prise amovibles pour l'accessoire (101) et un distributeur pour le produit (P).

10. Procédé de fabrication d'un conteneur au moyen de la machine selon l'une quelconque des revendications précédentes, comprenant les étapes suivantes :
- production d'un élément tubulaire (110) en matière plastique semi-fluide à l'aide de moyens de production ;
- insertion de l'élément tubulaire (110) dans des moyens de moulage (2) et dans des moyens de formation (3) dans une position ouverte ;
- insertion d'un accessoire (101) à l'aide de moyens de support (4), à l'intérieur de l'élément tubulaire (110), dans les moyens de formation (3) ;
- placement des moyens de formation (3) dans une position de fermeture respective formant le conteneur (100) avec une extrémité ouverte (102) et une extrémité fermée et incorporant l'accessoire (101) dans celui-ci ;
- retrait au moins partiel des moyens de support (4), par le fait de les détacher de l'accessoire (101) ;
- amenée du produit (P) dans le conteneur (100), par l'extrémité ouverte (102) de celui-ci, à l'aide de moyens de remplissage (5) ;
- retirer les moyens de remplissage (5) ;
- placement des moyens de moulage (2) dans une position de fermeture respective, fermant l'extrémité ouverte (102) du conteneur (100) ;
- attente de la consolidation de la matière de l'élément tubulaire (110) ;
- placement des moyens de moulage (2) et des moyens de formation (3) dans la position d'ouverture et retrait du conteneur (100) formé, **caractérisé en ce que** lesdits moyens de formation (3) sont actionnés l'un vers l'autre jusqu'à ladite position de fermeture, de façon à former ledit conteneur (100) avec l'extrémité ouverte pour le remplissage du produit (P), et à comprimer l'élément tubulaire (110), au niveau de l'extrémité fermée, directement sur une partie dudit accessoire (101) de façon à incorporer partiellement ledit accessoire (101) dans ledit conteneur, au niveau de l'extrémité fermée.

11. Procédé selon la revendication 10, **caractérisé en ce qu'**il sépare le conteneur formé (100) des restes de l'élément tubulaire (110).

12. Procédé selon la revendication 10, **caractérisé en ce qu'**il maintient la pression interne de l'élément tubulaire (110) à une pression supérieure à la pression externe, à l'aide de moyens de soufflage ou de moyens de dépression externes.

13. Procédé selon la revendication 12, **caractérisé en ce qu'**il réduit la pression interne de l'élément tubulaire (110) avant le placement des moyens de moulage (2) à la position de fermeture respective.

14. Conteneur (100) réalisé au moyen de la machine selon l'une quelconque des revendications 1 à 9, ou au moyen du procédé selon l'une quelconque des revendications 10 à 13, comprenant au moins une partie creuse, contenant un produit (P) à distribuer, et un accessoire (101) au moins partiellement incorporé dans la partie creuse et s'écoulant dans la partie creuse,
**caractérisé en ce qu'**une partie d'extrémité dudit accessoire (101) est incluse ou incorporée directement dans la paroi dudit conteneur (100) à travers la matière pastique comprimée dudit conteneur (100).

15. Conteneur (100) selon la revendication 14, **caractérisé en ce qu'**il comprend un organe de préhension (103) réalisé d'un seul tenant avec le conteneur (100) à l'extrémité dudit conteneur (100) qui est opposée à l'accessoire (101).

16. Conteneur (100) selon la revendication 15, **caractérisé en ce que** l'organe de préhension (103) a une forme presque rectangulaire et aplatie.

17. Conteneur (100) selon la revendication 14, **caractérisé en ce qu'**il comprend un moyen de protection (104) pour la partie de l'accessoire (101) qui dépasse de la partie destinée au produit (P).

18. Conteneur (100) selon la revendication 17, **caractérisé en ce que** le moyen de protection (104) est fixé à la partie restante du conteneur (100) par l'intermédiaire d'un moyen de séparation (105) du moyen de protection (104).

19. Conteneur (100) selon la revendication 18, **caractérisé en ce que** le moyen de séparation (105) a une ligne fermée au niveau de laquelle la matière du conteneur est amincie.

20. Conteneur (100) selon la revendication 17, **caractérisé en ce que** le moyen de protection (104) est solidaire de la partie restante du conteneur (100).

21. Conteneur (100) selon la revendication 14, **caractérisé en ce que** l'accessoire (101) est une aiguille pour injections.

22. Conteneur (100) selon la revendication 21, **caractérisé en ce que** la partie de l'aiguille (101) qui est incorporée dans la matière du conteneur (100) est pourvue d'au moins l'un des éléments parmi : des reliefs, des creux, des rainures, des moletages, et analogue.

23. Conteneur (100) selon l'une quelconque des revendications 17 à 21, **caractérisé en ce que** la partie du moyen de protection (104) correspondant à la pointe de l'aiguille (101), présente un gonflement (106).
